# EUROPÄISCHE PATENTANMELDUNG

(11) **EP 2 263 544 A1**
(43) Veröffentlichungstag der Anmeldung: **22.12.2010**
(21) Anmeldenummer: 09160555.0
(22) Anmeldetag: 18.05.2009
(51) Int. Cl.: A61B 5/151, A61B 5/1455, A61B 5/00

(54) **Testeinheit zur Untersuchung einer Körperflüssigkeit und Herstellungsverfahren**

(71) Anmelder: F. Hoffmann-La Roche AG, 4070 Basel (CH); Roche Diagnostics GmbH, 68305 Mannheim (DE)
(72) Erfinder: Die Erfindernennung liegt noch nicht vor
(74) Vertreter: Pfiz, Thomas

(57) **Zusammenfassung**

Die Erfindung betrifft eine Testeinheit zur Untersuchung einer Körperflüssigkeit mit einem in die Haut eines Benutzers einstechbaren Stechelement (12), das mit einem Sammelbereich (18) zur Aufnahme von Körperflüssigkeit versehen ist, und einem an dem Stechelement (12) angeordneten Nachweiselement (14), das einen an eine optische Messeinrichtung ankoppelbaren transparenten Träger (22) und ein darauf flächig aufgebrachtes analytisches Testfeld (24) aufweist. Erfindungsgemäß wird vorgeschlagen, dass eine mit dem Sammelbereich (18) fluidisch verbundene Messzone (32) des Testfelds (24) durch eine linienförmige Fließbarriere (30) begrenzt ist, wobei die Fließbarriere (30) einen Transport der Körperflüssigkeit in einen an die Messzone (32) angrenzenden Randbereich (34) des Testfelds (24) verhindert.

## Beschreibung

Die Erfindung betrifft eine Testeinheit zur Untersuchung einer Körperflüssigkeit mit einem in die Haut eines Benutzers einstechbaren Stechelement, das mit einem Sammelbereich zur Aufnahme von Körperflüssigkeit versehen ist, und einem an dem Stechelement angeordneten Nachweiselement, das einen vorzugsweise mittels Lichtleiter an eine optische Messeinrichtung ankoppelbaren transparenten Träger und ein darauf vollflächig aufgebrachtes analytisches Testfeld aufweist. Die Erfindung betrifft weiter ein Verfahren zur Herstellung einer solchen Testeinheit.

Analytische Systeme mit solchen Testeinheiten zur Bestimmung von Glukose in Blut sind beispielsweise aus der WO 2007/045412 der Anmelder bekannt. Dort sind die disposiblen Testeinheiten mit integrierten Lichtleitern versehen, um die reflexionsphotometrische Messung direkt in dem Sammelbereich ohne aufwändigen Probentransport zu ermöglichen, wobei das analytische Testfeld frontal beaufschlagt wird. Um eine sichere Funktion zu gewährleisten, muss dabei das gesamte Testfeld hinreichend benetzt werden.

Ausgehend hiervon liegt der Erfindung die Aufgabe zugrunde, die im Stand der Technik bekannten Testeinheiten weiter zu verbessern und insbesondere für die zuverlässige Verarbeitung kleinster Probenmengen zu optimieren.

Zur Lösung dieser Aufgabe wird die im Patentanspruch 1 bzw. 14 angegebene Merkmalskombination vorgeschlagen. Vorteilhafte Ausgestaltungen und Weiterbildungen der Erfindung ergeben sich aus den abhängigen Ansprüchen.

Die Erfindung geht von dem Gedanken aus, eine definierte Blutmenge auf einem begrenzten Flächenausschnitt des Testfelds bereitzustellen. Dementsprechend wird erfindungsgemäß vorgeschlagen, dass eine mit dem Sammelbereich fluidisch verbundene zentrale Messzone und eine umgebende Außenzone als Teilabschnitte bzw. Segmente des Testfelds durch eine linienförmige Fließbarriere voneinander getrennt sind, wobei die Fließbarriere einen Transport der Körperflüssigkeit aus der Messzone in die Außenzone beschränkt oder verhindert. Die Außenzone bildet somit einen zumindest weitgehend flüssigkeitsfreien Abschnitt und die Messzone einen flüssigkeitsbeaufschlagten Abschnitt in der Ebene des Testfelds. Dadurch ist es möglich, das Nachweiselement an geeigneten Strukturen zu montieren, ohne den Messbereich zu beeinflussen. Ein Fließtransport in der Ebene des Testfelds wird somit über die Fließbarriere hinaus unterdrückt. Durch die resultierende Beschränkung des Benetzungsbereichs kann sichergestellt werden, dass in der Messzone der notwendige Flüssigkeitsspiegel zuverlässig erreicht wird. Die hierfür erforderliche reduzierte Probenmenge ist vorteilhaft für eine hohe Erfolgsrate bei der Probengewinnung und insbesondere bei der Gewinnung von Blut und/oder Gewebeflüssigkeit aus der Haut. Dabei kann mit geringerer Stechtiefe und damit geringerem Stechschmerz mit höherer Wahrscheinlichkeit eine ausreichende Blutmenge gewonnen werden.

Vorteilhafterweise ist die Fließbarriere durch eine Schnittlinie gebildet, die sich vorzugsweise als Laserschnittlinie mit der erforderlichen Präzision herstellen lässt. Hierbei ist es günstig, wenn die Fließbarriere am vorgefertigten Nachweiselement durch ein Schneidwerkzeug, insbesondere ein Laserschneidwerkzeug von der freien Testfeldoberfläche her in das Material des Testfelds eingebracht wird.

Für eine auch hinsichtlich der Messwerterfassung optimierte Flächenbegrenzung ist es von Vorteil, wenn die Schnittlinie insbesondere als Kreis, Ellipse, Rechteck oder Raute in sich geschlossen ist.

Zur weiteren Verbesserung der Positionierung ist es vorteilhaft, wenn die Schnittlinie nach Maßgabe eines entsprechend dem Strahlengang der Messeinrichtung auf das Testfeld eingestrahlten Leuchtflecks zentriert ist.

Eine weitere vorteilhafte Ausgestaltung sieht vor, dass das Testfeld eine mit einem Inhaltsstoff der Körperflüssigkeit reagierende Nachweisschicht und eine die Nachweisschicht zu dem Sammelbereich hin überdeckende, mit der Fließbarriere versehene kapillaraktive Spreithilfe zur schichtförmigen Verteilung der Körperflüssigkeit aufweist. Damit ist es möglich, eine gewisse Mindestschichthöhe der Flüssigkeitsprobe in der Messzone zu gewährleisten.

In diesem Zusammenhang lässt sich eine weitere Verbesserung dadurch erreichen, dass das Testfeld eine poröse kapillaraktive Flachstruktur und insbesondere eine poröse Membran als Spreithilfe zur schichtförmigen Verteilung der Körperflüssigkeit aufweist.

Um die benötigte Flüssigkeitsmenge weiter zu reduzieren, ist es von Vorteil, wenn die poröse Flachstruktur eine asymmetrische Porengrößenverteilung über ihre Dicke besitzt, wobei eine grobporige Seite dem Sammelbereich zugewandt und eine feinporige Seite davon abgewandt ist. Dadurch wird eine zunehmende Kapillaraktivität in Richtung der Reagenzschicht erreicht, so dass die Spreithilfe nicht vollständig gefüllt sein muss, um eine hinreichende Benetzung zu gewährleisten.

Für eine zuverlässige Flüssigkeitsaufnahme ist es weiterhin günstig, wenn die Porengröße der porösen Flachstruktur in einem in mechanischem Kontakt zu dem Sammelbereich stehenden Anschlussbereich kleiner als die lichte Weite des Sammelbereichs ist.

Um eine ungewollte Verfälschung der Messergebnisse zu vermeiden, sollte die Spreithilfe die Körperflüssigkeit aus dem Sammelbereich unter Aufrechterhaltung einer Mindestschichthöhe vorzugsweise von 50 Mikrometer Flüssigkeitsspiegel schichtförmig über der Reagenzschicht verteilen.

Um die beim Einstich aufgenommene Probenflüssigkeit gezielt zu verwerten, ist es vorteilhaft, wenn der Sammelbereich durch einen Kanal, vorzugsweise einen Kapillarkanal gebildet ist, und wenn die Messzone einen proximalen Mündungsquerschnitt des Kanals begrenzt.

Vorteilhafterweise ist der Träger als Zuschnitt aus einer Folie gebildet und weist eine von dem Testfeld überdeckte Fläche von weniger als 5 mm², vorzugsweise weniger als 1 mm², beispielsweise 0,4 mm² auf. Hierbei ist es günstig, wenn die Flächenausdehnung der Messzone im Bereich zwischen 0,1 und 0,5 mm liegt.

Für die gleichzeitige optische und fluidische Ankopplung ist es vorteilhaft, wenn das Nachweiselement an dem Stechelement durch Verbindungsmittel fest fixiert ist, so dass eine rückseitige reflektometrische Vermessung bei vorderseitigem Kontakt zu dem Sammelbereich möglich ist.

Gegenstand der Erfindung ist auch ein Magazin mit einer Mehrzahl von erfindungsgemäßen Testeinheiten als Verbrauchsmittel zur Bestimmung von Blutglukose in einem Handgerät.

In verfahrensmäßiger Hinsicht wird die eingangs genannte Aufgabe dadurch gelöst, dass eine linienförmige Fließbarriere vorzugsweise als Schnittlinie in das Testfeld eingebracht wird, so dass die Fließbarriere eine mit dem Sammelbereich fluidisch verbundene Messzone gegenüber einer umgebenden Außenzone des Testfelds begrenzt. Vorteilhafterweise erfolgt die Positionierung dadurch, dass entsprechend dem Strahlengang einer zur Vermessung der Testeinheit ausgebildeten photometrischen Messeinrichtung ein Leuchtfleck auf das Testfeld eingestrahlt wird, und dass die in sich geschlossene Fließbarriere relativ zu dem Leuchtfleck positioniert, insbesondere zentriert wird.

Im Folgenden wird die Erfindung anhand eines in der Zeichnung schematisch dargestellten Ausführungsbeispiels näher erläutert. Es zeigen:
- Fig. 1: eine Testeinheit als Verbrauchsmittel zur Blut- glukosebestimmung in perspektivischer Ansicht;
- Fig. 2: die Testeinheit nach Fig. 1 bei angekoppelter optischer Messeinrichtung in ausschnittsweiser schaubildlicher Ansicht;
- Fig. 3: ein mit einer Fließbarriere versehenes Nach- weiselement der Testeinheit in perspektivischer Darstellung; und
- Fig. 4: ein Testsystem mit einem darin eingesetzten, teilweise aufgebrochen gezeigten Magazin für Testeinheiten in perspektivischer Darstellung.

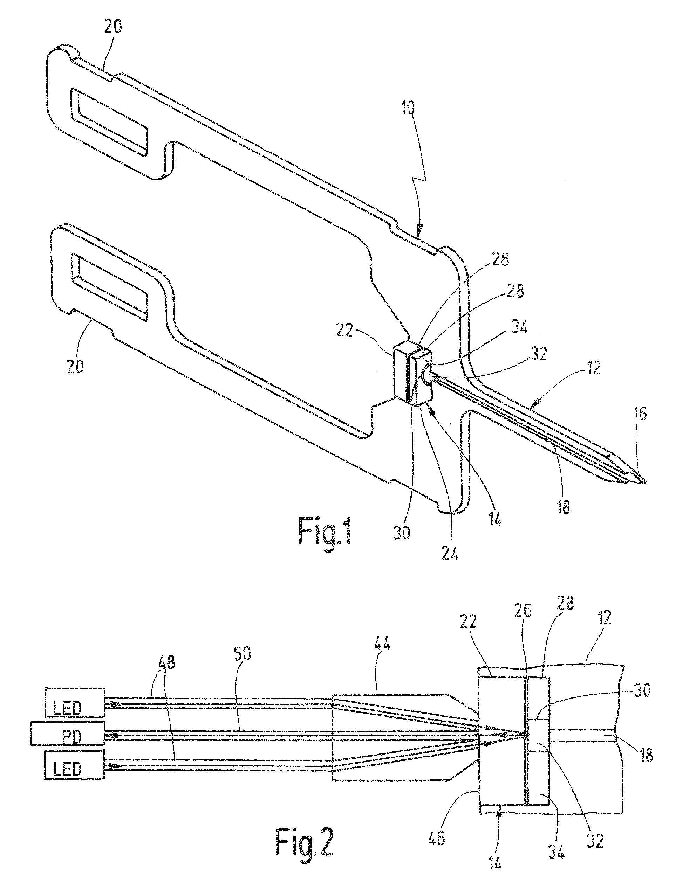

Die in Fig. 1 dargestellte Testeinheit 10 dient als analytisches Verbrauchsmittel zur Blutzuckermessung in einem Handgerät und besteht als so genannter Microsampler aus einem Stechelement 12 und einem daran fixierten Nachweiselement 14. Das Stechelement 12 ist als Flachformteil einstückig aus einem Edelstahlblech geätzt und weist ein nadelförmiges Stechorgan 16 auf, das mit einem rinnenförmigen Kapillarkanal 18 zur Blutaufnahme beim Hauteinstich versehen ist. Die beiden proximal abstehenden Arme 20 ermöglichen die Ankopplung eines geräteseitigen Stechantriebs für eine hin und her gehende Stechbewegung.

Wie aus Fig. 1 weiter zu ersehen ist, besteht das Nachweiselement 14 aus einem Träger 22 und einem Testfeld 24, das seinerseits aus einer Reagenzschicht 26 und einer Spreithilfe 28 aufgebaut ist. Die Spreithilfe 28 ist mit einer kreisförmigen Fließbarriere 30 versehen, um die Blutausbreitung auf eine zentrale Messzone 32 zu beschränken.

Der Träger 22 besteht aus einem transparenten Folienstück, das über geeignete Verbindungsmittel beispielsweise durch eine Klebe- oder Klemmverbindung in der Basis des Stechelements 12 fixiert ist. An seiner dem Kanal 18 zugewandten Vorderseite ist das Folienstück mit der Reagenzschicht 26 beschichtet, die als an sich bekanntes enzymatisches System auf einen Analyten (Glucose) in der Blutflüssigkeit irreversibel durch eine Farbänderung reagiert, die rückseitig durch das Folienstück hindurch reflektometrisch erfassbar ist. Da die Reagenzschicht 26 die Probenflüssigkeit nicht flächig verteilen kann, ist die Spreithilfe 28 als Flachstruktur so auf die Reagenzschicht 26 aufgebracht, dass sich eine Flüssigkeitsschicht mit einer Mindestschichthöhe zumindest über die Messzone 32 ausbildet. Hierbei ist die Flachstruktur 28 quer zu dem Kanal 18 ausgerichtet, wobei die Messzone 32 den proximalen Mündungsquerschnitt des Kanals 18 überdeckt. Denkbar ist es jedoch auch, dass die Messzone von dem Mündungsquerschnitt beabstandet ist und durch eine fluidische Verbindung mit Körperflüssigkeit beaufschlagt wird. Um den gewünschten Flüssigkeitsspiegel auch bei beschränktem Probenvolumen aus dem Kanal 18 zu erhalten, ist ein Randbereich bzw. eine Außenzone 34 der Spreithilfe 28 durch die Fließbarriere 30 gegen eine Flüssigkeitsausbreitung abgeschirmt.

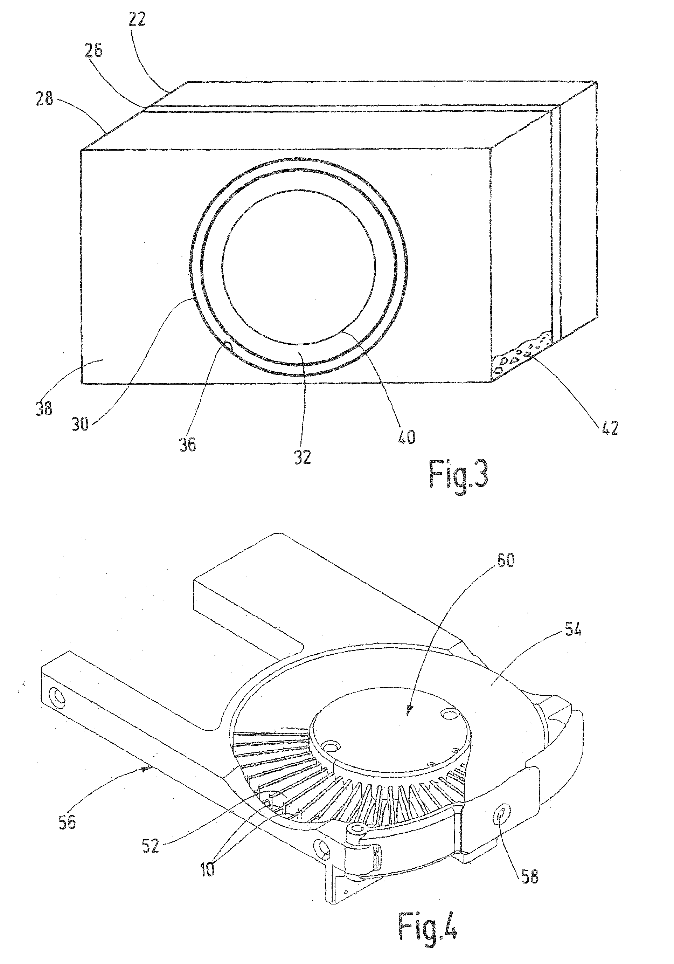

Wie am besten aus Fig. 3 ersichtlich, kann die Fließbarriere 30 durch einen kreisförmig umlaufenden Einschnitt 36 gebildet sein, der als Laserschnittspalt mittels eines CO₂-Lasers von der freien Vorderseite 38 der Spreithilfe 28 her eingebracht wird. Zu diesem Zweck kann entsprechend dem Strahlengang der Messeinrichtung durch rückseitige Einstrahlung ein Leuchtfleck 40 erzeugt werden, der durch ein Bildverarbeitungssystem erfasst wird, um den Laserstrahl so zu steuern, dass eine geeignete (koaxiale) Zentrierung der Fließbarriere 30 erreicht wird. Zweckmäßig wird für die Erzeugung des Leuchtflecks 40 eine Wellenlänge verwendet, welche eine Veränderung der Reaktionsschicht 26 weitgehend ausschließt. Die Position der Fließbarriere 30 kann aber auch ausgehend von den Rändern des Testfeldes 24 oder der Position von anderen Elementen der Testeinheit 10 abgeleitet werden. Der Laserschnitt muss die Spreithilfe 28 nicht vollständig durchdringen und kann auch zumindest teilweise als Schmelzlinie ausgebildet sein. Wichtig ist, dass die so gebildete die Fließbarriere einen Kapillarfluss der Körperflüssigkeit mindestens weitgehend unterbricht, so dass kein für die Messung relevanter Flüssigkeitsverlust auftritt.

Wie in Fig. 3 weiter veranschaulicht, ist die Spreithilfe 28 durch eine kapillaraktive poröse Membran 42 gebildet, die eine asymmetrische Porengrößenverteilung über ihre Dicke besitzt, wobei eine grobporige Seite in mechanischem Kontakt zu dem Kanal 18 steht und eine feinporige Seite auf der Reagenzschicht 26 sitzt. Dabei sollte die kanalseitige Porengröße kleiner als die lichte Weite des Kanals 18 sein. Beispielsweise kann die Grobporengröße 20 bis 60 Mikrometer und die Feinporengröße 0,1 bis 2 Mikrometer betragen. Ein solches Folienmaterial aus Polyethersulfon (PESu hydrophiliert mit Hydroxypropylcellulose) ist kommerziell erhältlich.

Sobald im Kanal 18 beim Hauteinstich aufgenommenes Blut durch Kapillarwirkung antransportiert wird und der Meniskus die Kanalmündung erreicht, kommt das Blut in Kontakt mit dem kapillaraktiven Material und wird eingesaugt. Solange das poröse Material 42 ausreichende Benetzbarkeit zeigt und die Reagenzschicht 26 in innigem Kontakt mit dem porösen Material steht, wird auch die Reagenzschicht mit Blut benetzt. Ausreichende Benetzbarkeit ist gegeben, wenn die Probeflüssigkeit in das poröse Material 42 gesaugt wird. Inniger Kontakt bedeutet, dass zwischen den festen Teilen des porösen Materials 42 und der Oberfläche der Reagenzschicht 26 keine Zwischenräume auftreten, die größer sind als die größten Poren des porösen Materials, und dass in Abständen, die kleiner sind als die Durchmesser der größten Poren des porösen Materials, punktuell direkter mechanischer Kontakt besteht.

Zweckmäßig beträgt die Gesamtfläche der freien Vorderseite 38 der Spreithilfe 28 etwa 0,4 mm², während der Durchmesser der Messzone 32 im Bereich zwischen 0,1 bis 0,5 mm liegt. Eine hinreichende Schichthöhe der Probenflüssigkeit von 50 Mikrometer kann dann bereits mit einem Probenvolumen von 4 bis 6 Nanoliter erreicht werden. Bei geringeren Schichthöhen beeinflusst ansonsten die Schichthöhe selbst das Messsignal.

Fig. 2 veranschaulicht den Strahlengang bei der reflektometrischen Vermessung des über den Kanal 18 mit Blut beaufschlagten Nachweiselements 14. Hierzu wird ein geräteseitiger Optikadapter 44 an die freie Rückseite 46 der Trägerfolie 22 angedockt. Das Licht von zwei Leuchtdioden LED mit ggf. unterschiedlicher Wellenlänge wird über zugeordnete Lichtleiter 48 in den Bereich der Messzone 32 eingestrahlt. Das in der Reagenzschicht 26 gestreute Messlicht wird ebenfalls über einen zugeordneten mittigen Lichtleiter 50 auf eine Photodiode PD als Detektor zurückgeleitet.

Wie in Fig. 4 gezeigt, lässt sich eine Vielzahl von Testeinheiten 10 in jeweiligen Magazinkammern 52 eines Scheibenmagazins 54 bereithalten, um in einem Handgerät 56 für einen Benutzer die Durchführung von Selbsttests zu ermöglichen. Die einzelnen Testeinheiten 10 sind dabei durch Drehung des Magazins 54 sukzessive in eine aktive Gebrauchsposition bezüglich einer mit einer Durchstechöffnung versehenen Auflage 58 zur Fingerpositionierung bringbar. Ein in die aktiv positionierte Magazinkammer 52 eingreifende Stechantrieb 60 ermöglicht eine Stechbewegung. Nach erfolgter Blutaufnahme und Messwerterfassung kann die gebrauchte Testeinheit 10 in der zugehörigen Magazinkammer 52 entsorgt werden. Dadurch wird die Selbstbestimmung der Blutzuckerkonzentration in einem vollautomatischen Messablauf auch für Laien mit hohem Handhabungskomfort zuverlässig durchführbar.

## Patentansprüche

1. Testeinheit zur Untersuchung einer Körperflüssigkeit mit einem in die Haut eines Benutzers einstechbaren Stechelement (12), das mit einem Sammelbereich (18) zur Aufnahme von Körperflüssigkeit versehen ist, und einem an dem Stechelement (12) angeordneten Nachweiselement (14), das einen vorzugsweise mittels Lichtleiter (48,50) an eine optische Messeinrichtung ankoppelbaren transparenten Träger (22) und ein darauf flächig aufgebrachtes analytisches Testfeld (24) aufweist, **dadurch gekennzeichnet, dass** eine mit dem Sammelbereich (18) fluidisch verbundene Messzone (32) von einer umgebenden Außenzone (34) des Testfelds (24) durch eine linienförmige Fließbarriere (30) getrennt ist, wobei die Fließbarriere (30) einen Transport der Körperflüssigkeit aus der Messzone (32) in die Außenzone (34) beschränkt oder verhindert.

2. Testeinheit nach Anspruch 1, **dadurch gekennzeichnet, dass** die Fließbarriere (30) durch eine Schnittlinie, vorzugsweise durch eine Laserschnittlinie gebildet ist.

3. Testeinheit nach Anspruch 1 oder 2, **dadurch gekennzeichnet, dass** die Fließbarriere (30) durch ein Schneidwerkzeug von der freien Testfeldoberfläche (38) her in das Material des Testfelds (24) eingebracht ist.

4. Testeinheit nach einem der Ansprüche 1 bis 3, **dadurch gekennzeichnet, dass** die linienförmige Fließbarriere (30) insbesondere als Kreis, Ellipse, Rechteck oder Raute in sich geschlossen ist.

5. Testeinheit nach einem der Ansprüche 1 bis 4, **dadurch gekennzeichnet, dass** das Testfeld (24) eine mit einem Inhaltsstoff der Körperflüssigkeit reagierende Nachweisschicht (26) und eine die Nachweisschicht (26) zu dem Sammelbereich (18) hin überdeckende, mit der Fließbarriere (30) versehene kapillaraktive Spreithilfe (28) zur schichtförmigen Verteilung der Körperflüssigkeit aufweist.

6. Testeinheit nach einem der Ansprüche 1 bis 5, **dadurch gekenntzeichnet, dass** das Testfeld (24) eine poröse Flachstruktur (42), insbesondere eine poröse Membran als Spreithilfe (28) zur schichtförmigen Verteilung der Körperflüssigkeit aufweist.

7. Testeinheit nach Anspruch 6, **dadurch gekennzeichnet, dass** die poröse Flachstruktur (42) eine asymmetrische Porengrößenverteilung über ihre Dicke besitzt, wobei eine grobporige Seite dem Sammelbereich (18) zugewandt und eine feinporige Seite davon abgewandt ist.

8. Testeinheit nach Anspruch 6 oder 7, **dadurch gekennzeichnet, dass** die Porengröße der porösen Flachstruktur (42) in einem in mechanischem Kontakt zu dem Sammelbereich (18) stehenden Anschlussbereich kleiner als die lichte Weite des Sammelbereichs (18) ist.

9. Testeinheit nach einem der Ansprüche 6 bis 8, **dadurch gekennzeichnet, dass** die Spreithilfe (28) die Körperflüssigkeit aus dem Sammelbereich (18) unter Aufrechterhaltung einer Mindestschichthöhe vorzugsweise von 50 Mikrometer Flüssigkeitsspiegel schichtförmig verteilt.

10. Testeinheit nach einem der Ansprüche 1 bis 9, **dadurch gekennzeichnet, dass** der Sammelbereich (18) durch einen Kanal, vorzugsweise einen Kapillarkanal gebildet ist, und dass die Messzone (32) einen proximalen Mündungsquerschnitt des Kanals begrenzt.

11. Testeinheit nach einem der Ansprüche 1 bis 10, **dadurch gekennzeichnet, dass** der Träger (22) als Zuschnitt aus einer Folie gebildet ist und eine von dem Testfeld (24) überdeckte Fläche von weniger als 5 mm², vorzugsweise weniger als 1 mm² aufweist.

12. Testeinheit nach einem der Ansprüche 1 bis 11, **dadurch gekennzeichnet, dass** die Flächenausdehnung der Messzone (32) im Bereich zwischen 0,1 und 0,5 mm liegt.

13. Testeinheit nach einem der Ansprüche 1 bis 12, **dadurch gekennzeichnet, dass** das Nachweiselement (14) an dem Stechelement (12) durch Verbindungsmittel fest fixiert ist, so dass eine rückseitige reflektometrische Vermessung bei vorderseitigem Kontakt zu dem Sammelbereich (18) möglich ist.

14. Verfahren zur Herstellung einer Testeinheit nach einem der vorhergehenden Ansprüche, bei welchem ein in die Haut eines Benutzers einstechbares und einen Sammelbereich (18) zur Aufnahme von Körperflüssigkeit aufweisendes Stechelement (12) mit einem Nachweiselement (14) versehen wird, wobei das Nachweiselement aus einem transparenten Träger (22) und einem darauf flächig aufgebrachten analytisches Testfeld (24) gebildet wird, **dadurch gekennzeichnet, dass** eine linienförmige Fließbarriere (30) in das Testfeld (24) eingebracht wird, so dass die Fließbarriere (30) eine mit dem Sammelbereich (18) fluidisch verbundene Messzone (32) gegenüber einer umgebenden Außenzone (34) des Testfelds (24) begrenzt.

15. Verfahren nach Anspruch 14, **dadurch gekennzeichnet, dass** entsprechend dem Strahlengang einer zur Vermessung der Testeinheit ausgebildeten photometrischen Messeinrichtung ein Leuchtfleck (40) auf das Testfeld (24) eingestrahlt wird, und dass die in sich geschlossene linienförmige Fließbarriere (30) relativ zu dem Leuchtfleck positioniert, insbesondere zentriert wird.
